# EUROPEAN PATENT APPLICATION

(11) **EP 1 398 044 A1**
(43) Date of publication of application: **17.03.2004**
(21) Application number: 03005486.0
(22) Date of filing: 17.03.2003
(51) Int. Cl.: A61L 9/12, A61L 9/04

(54) **Method and apparatus for releasing fragrance**

(30) Priority: 09.09.2002 US 237115
(71) Applicant: The Egg Factory L.L.C., Roanoke, VA 24017 (US)
(72) Inventor: Benio, Kevin, Herndon, Virginia 20171-3527 (US); Skiles, Joshua, Pilot, Virginia 24138 (US); Sterner, Shannon, Charlottesville, Virginia 22904 (US); Teitt, Sarah, Marysville, Ohio 43040 (US); Gentiluomo, Andrew, Roanoke, Virginia 24018 (US)
(74) Representative: VOSSIUS & PARTNER

(57) **Abstract**

A method and apparatus for releasing fragrance. An embodiment of the apparatus includes a first scented layer that releases a first portion of fragrance and a second scented layer adjacent to the first layer that releases a second portion of fragrance. The second portion of fragrance is released after the first portion of fragrance is released. Exemplary applications include air fresheners and fragrance samplers.

## Description

This application claims priority to U.S. provisional application no. 60/317,485, filed September 7, 2001, the entire contents of which are incorporated herein by reference.

Embodiments of the present invention relate to fragrance releasing devices and, more particularly, to a method and an apparatus for releasing fragrance.

Air fresheners are fragrance releasing devices that are generally used to disguise strong odors or establish pleasant aromas in buildings, vehicles, and other enclosed areas. Air fresheners generally contain some form of moist fragrant substance that evaporates to produce an airborne scent. The scent is generally released either quickly or slowly, but not both. A problem with these air fresheners is that they generally emit a strong concentration of fragrance initially, but then steadily decrease to a very weak or undetectable concentration over time. So, the initial concentration of fragrance is very difficult to control or maintain over the life of the air freshener.

Some air fresheners are designed to allow the user to control the amount of fragrant substance released and, hence, control the fragrance strength. However, these user-controlled air fresheners are usually powered by electricity, for example, which requires that they take up electrical outlet space and be limited in where they can be placed in a room. Such placement may be inconvenient. Additionally, the use of these air fresheners is limited to areas having electrical outlets. So, automobiles, closets, etc., are not viable for placement.

Fragrance samplers for cologne and perfume are fragrance releasing devices that are generally used for advertising and testing products. Samplers generally include either a small thick paper card on which fragrance is applied or sample vials in which liquid fragrance is contained. Sample cards are generally given out to consumers in retail stores to advertise a particular product. However, a problem with these sample cards is that the fragrance on the card lasts only a short amount of time and is not transferable to one or more consumers' skin, for example, thereby limiting the effectiveness of the card as an advertising medium. Sample vials are also generally given out to consumers in retail stores. While the vials retain fragrance better than the sample cards, the vials present handling problems, e.g., the vials may be breakable or not shaped for carrying, for example, in a purse, pocket, or wallet, or the sample may likely be spilled or not easily transferable to one or more consumer.

Sample cards are also included in magazines or other paper media. These sample cards generally contain one sample of microencapsulated fragrance for a single use. The fragrance is released when the micro capsules are broken open. However, the microencapsulation does not allow the scent to be removed and transported mostly intact from the medium. Thus, the scented advertisement is limited to the readers of the paper media. As such, these samplers do not provide a versatile and simple method for incorporating scents into an advertisement such that the scent is removable from the printed material, transportable and able to be used as a sample for one or more consumer's personal needs.

### Brief Description of the Drawings

Fig. 1 is a top view of an embodiment of the present invention;
Fig. 2 is a side view of an embodiment of the present invention;
Fig. 3 is a side view of an embodiment of components of the present invention;
Fig. 4 is a cross-sectional front view of an embodiment of the present invention;
Fig. 5 is a cross-sectional front view of another embodiment of the present invention;
Fig. 6 is a cross-sectional front view of another embodiment of the present invention;
Fig. 7 is a cross-sectional front view of yet another embodiment of the present invention;
Fig. 8 is a cross-sectional front view of another embodiment of the present invention;
Fig. 9 is an illustration of an embodiment of the present invention in use;
Fig. 10 is an illustration of an exemplary container which may house an embodiment of the present invention;
Fig. 11 is an illustration of a side view of the exemplary container of Fig. 9;
Fig. 12 is a side view of a rolled embodiment of the present invention;
Fig. 13 is a method for releasing fragrance according to an embodiment of the present invention; and
Fig. 14 is an illustration of exemplary fragrance concentrations released by an embodiment of the present invention.

### Detailed Description

Embodiments of the present invention include a method and an apparatus for releasing fragrance. These embodiments may advantageously provide controlled fragrance release, ranging from short to long duration, unconstrained placement, easy replacement, and customization. In one embodiment of an apparatus of the present invention, the apparatus may include a first scented layer and a second scented layer, in which the first and second scented layers release respective first and second portions of fragrance. In one embodiment of a method of the present invention, the method may include releasing a first portion of fragrance in the first layer, and releasing a second portion of fragrance in the second layer, where the second portion is released after the first portion.

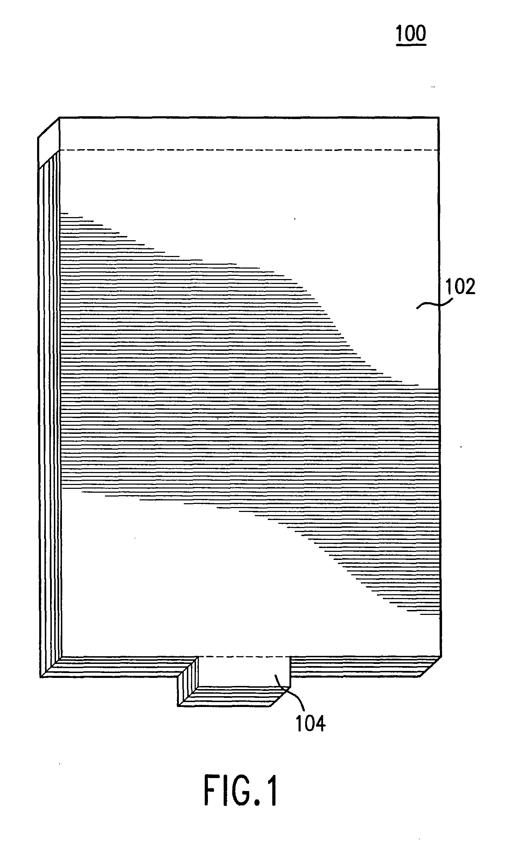

Figure 1 is a top view of an embodiment of a fragrance releasing apparatus of the present invention. Apparatus 100 may include a stack of fragrance releasing units 102 and tabs 104 attached to each unit. Units 102 may include fragrance to be released. Tabs 104 may be used to remove layers of units 102 during fragrance release. Exemplary applications of apparatus 100 include an air freshener, a fragrance sampler, and a fragrance applicator or storage unit.

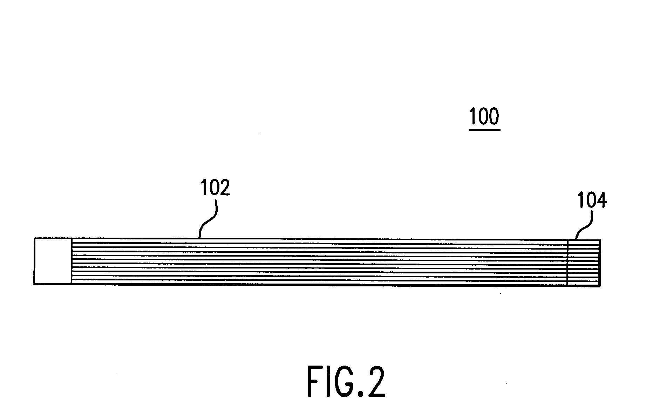

Figure 2 is a side view of an embodiment of a fragrance releasing apparatus of the present invention. The stack of fragrance releasing units 102 and tabs 104 are shown.

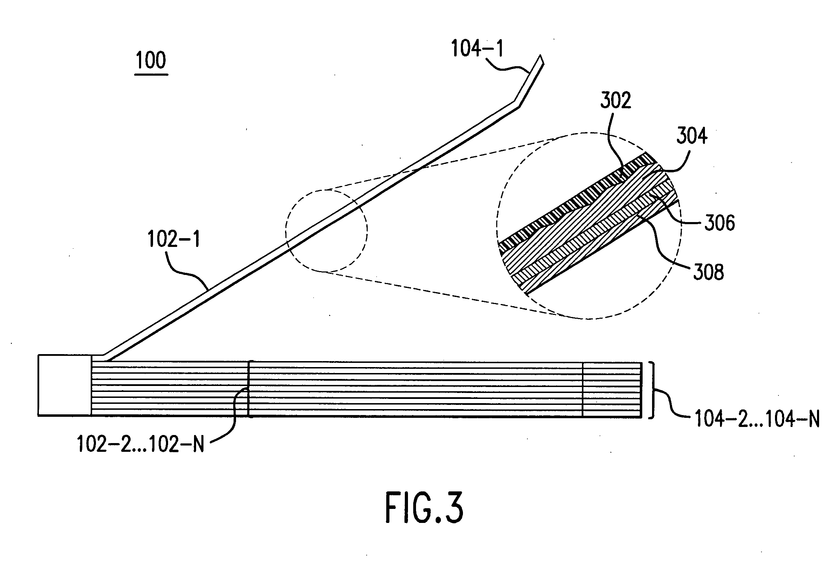

Figure 3 is a side view of an embodiment of a fragrance releasing apparatus of the present invention, including components of one of the units. Fragrance releasing unit 102-1 may be removed from remaining stacked units 102-2...102-N using tab 104-1. Tabs 104-2...104-N may be used to separate their respective units 102-2...102-N in a similar manner. Fragrance releasing unit 102-1 may include multiple layers as shown in Figure 3. The layers may include a top sealant layer 302, a first scented layer 304, and a second scented layer 306. In an alternate embodiment, the layers may include a bottom sealant layer 308 which will be described later. Top sealant layer 302 may include tab 104-1. Top sealant layer 302 may seal scented layers 304, 306 in order to preserve their scent until the consumer is ready to use apparatus 100. Sealant layer 302 may cover scented layers 304, 306 and be removed from scented layers 304, 306 using tab 104-1. Sealant layer 302 may be made of plastic, by way of example only, polyethylene or polyvinylidene chloride (PVDC) films, plastic-like material, polymer, polymer-like material, modified paper sheets or any other material capable of sealing in at least a liquid, gel (or gelatin), solid, and/or some other substance form. Layer 302 may be biodegradable. Layer 302 may be scented.

First scented layer 304 may release a portion of fragrance from apparatus 100. Layer 304 may be an aroma-containing liquid, gel, liquid-gel combination, or solid. Second scented layer 306 may release another portion of fragrance from apparatus 100 after first layer 304 has been depleted. Layer 306 may be an aroma-containing gel or solid. Layers 304, 306 may be fast-, moderate-, or slow-releasing depending on the specific application and layer composition. For example, a liquid layer may be fastest in releasing fragrance due to the rapid evaporation rate of a liquid. A gel may be slower in releasing fragrance because of the slower evaporation rate of a gel. And a solid may be the slowest of all because a solid evaporates very slowly relative to other forms of matter.

Exemplary chemical compounds making up scented layers 304, 306 may include, but are not limited to, alcohols, aliphatic alcohols, aldehydes, aliphatic aldehydes, acetaldehydes, paraldehydes, acroleins, glycerols, and any material suitable for containing fragrance and/or transferring fragrance to a consumer, e.g., a consumer's skin. An alcohol or glycerol layer may provide a fast release of fragrance for a short time. The volatility of the alcohol or glycerol may determine the extent of fragrance persistence or longevity. A gel layer may provide lower volatility and a solid layer lower still, providing a longer lasting, more persistent fragrance with slower release. Gel layers may include polymer geis formed by inflating polymer networks with solvent or water and include, by way of example only, gels formed from polyvinylalcohol (PVA), polyacrylicacid (PAA), and polyacrylonitrile (PAN).

It may be understood that a scented layer may also be in gas form. In this case, fragrance depletion may be immediate upon removing top sealant layer 302.

Stacked units 102 may provide for continuous and long use of the fragrance releasing apparatus. After one unit 102 is depleted, another one may be engaged. For example, after a desired amount of fragrance has been released from top unit 102-1 or after a desired release duration, top unit 102-1 may be removed from apparatus 100, revealing next unit 102-2 underneath. Then, next unit 102-2 may begin releasing fragrance by removing the next unit's top sealant layer 302. After next unit 102-2 releases a desired amount of fragrance or after a desired duration, next unit 102-2 may be removed from apparatus 100 and third unit 102-3 may begin releasing fragrance and so on until fragrance has been released from all stacked units 102. This stacking configuration may advantageously allow the consumer to regulate the strength of the scent throughout the estimated duration of the fragrance releasing apparatus. Consumers may remove top sealant layer 302 at their discretion, thus allowing consumers to easily control the fragrance and maintain a potent scent during a desired duration by easily replacing depleted units 102. Accordingly, apparatus 100 may overcome the control problem associated with some known fragrance releasing devices.

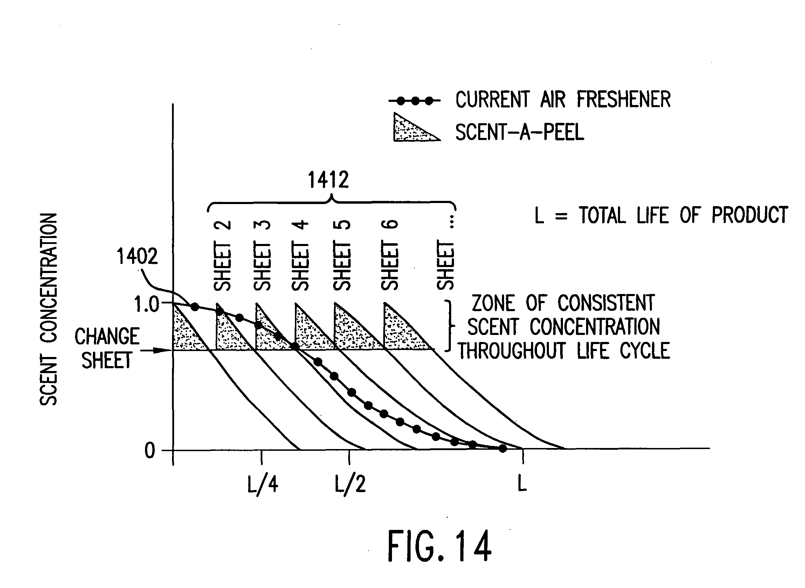

Figure 14 illustrates exemplary fragrance concentrations of an embodiment of the present invention compared to some known fragrance releasing devices. For a time period L, the fragrance concentration 1402 of some known fragrance releasing devices begins strong and gradually decreases to zero at time L, such that the fragrance eventually becomes undetectable. During this time period L, the consumer has no way of controlling the fragrance concentration. As such, the known device soon becomes ineffective.

On the other hand, for the same time period L, an embodiment of the present invention maintains a strong fragrance concentration 1412 for the entire time period. In this case, top sealant layer 302 may be removed from the topmost unit 102-1, strong concentrations of fragrance may then be released from scented layers 304, 306. As the fragrance concentration decreases, the consumer may then remove top sealant layer 302 of the next unit 102-2. Strong concentrations of fragrance may again be released from scented layers 304, 306 of the next unit 102-2. As the fragrance concentration again decreases, the consumer may then remove top sealant layer 302 of the third unit 102-3 and so on. As a result, the fragrance concentration does not fall below a desired value, such that the consumer may advantageously be able to maintain and control a strong, persistent fragrance throughout the entire duration of the embodiments of the present invention.

Sealant layer 302 may advantageously prevent spillage of a liquid scented layer. Accordingly, apparatus 100 may be easily transported, overcoming handling problems associated with some known fragrance releasing devices.

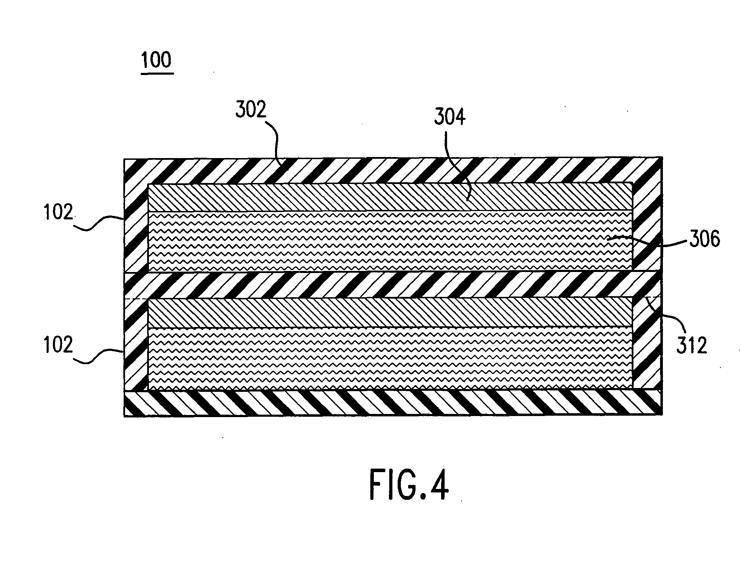

Figure 4 is a cross-sectional front view of an embodiment of a fragrance releasing apparatus according to the present invention. Apparatus 100 shows two exemplary stacked units 102. Apparatus 100 is not limited to two stacked units, but may include any number. Unit 102 may include a sealant layer 302, a first scented layer 304, and a second scented layer 306. In this embodiment, sealant layer 302 may cover scented layers 304, 306 both above and on the sides of unit 102. The sealant layer of the unit directly underneath, with the exception of the bottom-most unit, may provide a bottom sealant. As the last unit, the bottom-most unit may have a separate bottom sealant layer to help seal in its first and second layers. First scented layer 304 may include a substance with a higher volatility, e.g., an alcohol or substance of similar volatility, and second scented layer 306 may include a substance with a lower volatility, e.g., a gel or substance of similar volatility. First layer 304 may be thinner than second layer 306. As such, first layer 304 may release fragrance quickly and then second layer 306 may slowly release fragrance. The effect may be an immediate burst of scent followed by strong, longer lasting scent to permeate a large area. An exemplary application of this embodiment may be an air freshener.

In this embodiment, first scented layer 304 is thinner than second scented layer 306. However, it may be understood that the ratio of layer thickness may vary according to the nature of the fragrance product and the desired effect of the fragrance. It may be further understood that the fragrance in each layer may be the same or different according to the desired effect.

In the embodiment of Figure 4, sealant layer 302 may be removed from apparatus 100, exposing first layer 304 to air. First layer 304 may quickly release fragrance through evaporation of layer 304, for example. After first layer 304 is depleted, second layer 306 may be exposed to air. Second layer 306 may then slowly release fragrance through evaporation of layer 306, for example. After second layer 306 is depleted, the sealant layer of the unit underneath may be exposed to air. This unit's sealant layer may then be removed and the fragrance release process repeated for this unit. Hence, for stacked units, fragrance may continuously be released.

Optionally, sealant layer 302 may have perforated edges 312 such that when sealant layer 302 is removed from unit 102, only the top of sealant layer 302 may be removed. Hence, the sides of sealant layer 302 may remain in place and provide containment for first and second layers 304, 306. In this case, after first and second layers 304, 306 are depleted, the sides of sealant layer 302 may be removed with the next sealant layer.

Alternatively, apparatus 100 may include a bottom sealant layer 308, as shown in Figure 3. Bottom sealant layer 308 may seal scented layers 304, 306 underneath and act as an additional barrier to prevent premature fragrance release and to separate adjacent units 102. When scented layers 304, 306 are being released, layer 308 may also act as their container. Sealant layer 308 may be removed from apparatus 100 using a portion of tab 104. Sealant layer 308 may be made of plastic, plastic-like material, polymer, polymer-like material, modified paper sheets or any other material capable of sealing in at least a liquid, gel, solid, and/or some other substance form. Layer 308 may also be biodegradable. Layer 308 may be scented.

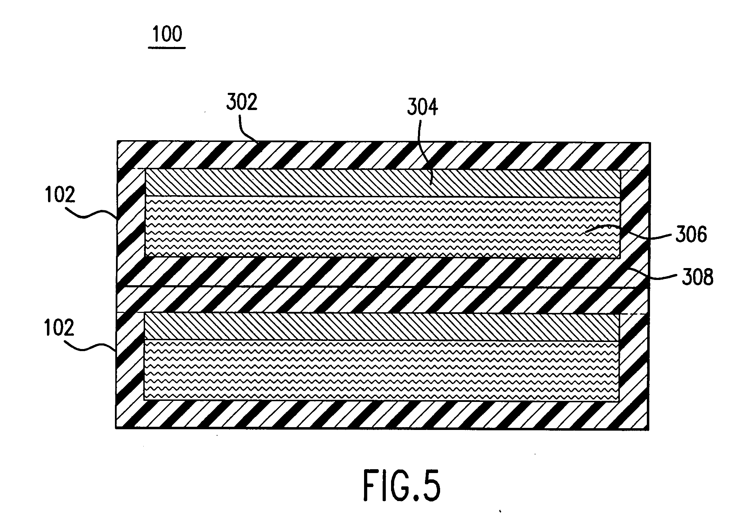

Figure 5 is a cross-sectional front view of an alternate embodiment of a fragrance releasing apparatus according to the present invention. In this embodiment, apparatus 100 may include bottom sealant layer 308 in addition to the layers described in Figure 4.

In alternate embodiments of a fragrance releasing apparatus, it may be understood that any number of scented layers with different thicknesses and/or volatilities may be included in the apparatus.

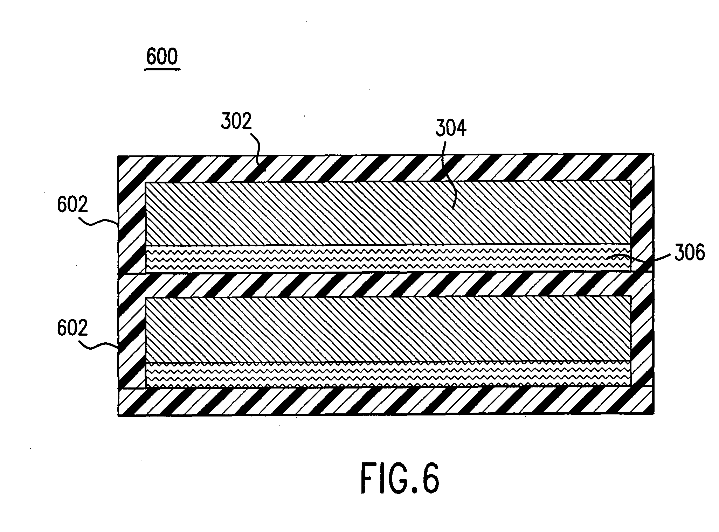

Figure 6 is a cross-sectional front view of another embodiment of a fragrance releasing apparatus according to the present invention. Apparatus 600 shows two exemplary stacked units 602. However, any number of units 602 may be included. Unit 602 may include a sealant layer 302, a first scented layer 304, and a second scented layer 306. In this embodiment, sealant layer 302 may encapsulate scented layers 304, 306 both above and on the sides of unit 602. The sealant layer of the unit directly underneath may provide a bottom sealant, with the exception of the bottom-most unit, which may include a bottom sealant layer. First scented layer 304 may include a substance with a higher volatility, e.g., an alcohol or similar volatile substance, and second scented layer 306 may include a substance with a lower volatility, e.g., a gel or similar volatile substance. Second layer 306 may be thinner than first layer 304. As such, first layer 304 may release fragrance quickly, but in larger amounts, and then second layer 306 may slowly release fragrance, but in small amounts, after first layer 304 is depleted. The effect may be a strong immediate scent followed by a weaker persistent fragrance that permeates only a small close area. An exemplary application of this embodiment may be a fragrance sampler, in which a consumer may apply the strong scent to the skin and smell the scent at close range rather than notice the scent in a large area.

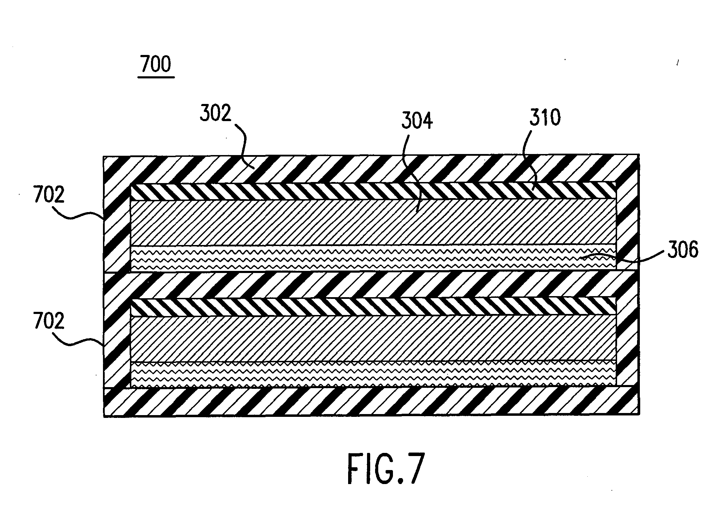

Figure 7 is a cross-sectional front view of another alternative embodiment of a fragrance releasing apparatus according to the present invention. Apparatus 700 shows two exemplary stacked units 702. However, apparatus 700 may include any number of units 702. Unit 702 may include a sealant layer 302, a first scented layer 304, a second scented layer 306, and an applied scent layer 310. Layer 302, 304, and 306 may perform similar to those described for Figure 6. Applied scent layer 310 may include a highly volatile substance, e.g., an alcohol or substance of similar volatility, and be thinner than layers 304, 306. As such, applied layer 310 may release fragrance immediately, followed by layers 304, 306. The effect may be a quick burst of scent followed by a strong immediate scent followed by a weaker persistent fragrance that permeates only a small close area. An exemplary application of this embodiment may be a fragrance product, in which a consumer may get an immediate scent, then apply the strong scent to the skin, and smell the scent at close range rather than notice the scent in a large area.

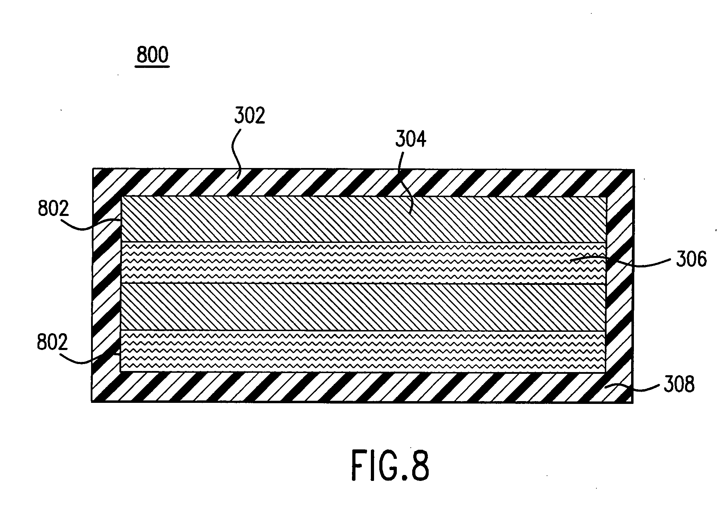

Figure 8 is a cross-sectional front view of another alternative embodiment of a fragrance releasing apparatus according to the present invention. Apparatus 800 shows two stacked units 802. However, apparatus 800 may include any number of units 802. In this embodiment, unit 802 may include first and second scented layers 304, 306. Top and bottom sealant layers 302, 308 may be omitted. As such, first and second scented layers 304, 306 may include substances with low volatility, e.g., geis or solids, which may be viscous and easily contained. Fragrance may be released slowly from first layer 304 and then slowly from second layer 306. Alternatively, apparatus 800 may include a top sealant layer 302 to cover the topmost first layer and a bottom sealant layer 308 to cover the sides of all the stacked units 802 and the bottom of the bottommost second layer. As such, less volatile and viscous scented layers may be used and contained by top and bottom sealant layers 302, 308.

In an alternate embodiment of the present invention, first layer 304 may be omitted or may be an outer superior surface of second layer 306, e.g., a gel layer.

Other exemplary applications include aromatherapy, disinfectants, and odor-eliminating devices. For example, the scented layers may include aromatherapy compositions, which may be released into a room, or antibacterial compounds, which may be applied to a skin cut or inhaled, or fragrances which may be released to neutralize odors.

In an embodiment of the present invention, bottom sealant layer 308 may change color after much or all fragrance has been released from first and second scented layers 304, 306. Alternatively, if bottom sealant layer 308 is absent, top sealant layer 302 of the next stacked unit may change color after much or all fragrance of the previous unit's first and second layers 304, 306 has been released. This color change may advantageously provide a convenient indicator to the consumer to begin fragrance release of the next stacked unit, thereby creating continuous, persistent scent.

In another embodiment, apparatus 100 may be customized to include any set of graphics, colors, color intensities, e.g., glow in the dark, shapes, sizes, models, and other forms or messages. This customization may be provided in sealant layers 302, 308 and/or scented layers 304, 306. For example, scented layer 304 may be a green-colored liquid and scented layer 306 underneath may be a yellow-calmed solid with a message or advertisement embedded therein. This customization may advantageously provide an aesthetic product for the consumer and, in the case of embedded messages, an effective advertising medium for manufacturers, distributors, retailers, etc., of apparatus 100. Additionally, different colors for each layer may advantageously provide consumers with another convenient indicator of when a scented layer is depleted. Similarly, sealant layers 302, 308 may have graphics, messages, advertisements, etc., printed on them.

Accordingly, embodiments of the present invention may advantageously provide a combination of control, fragrance longevity, and customization.

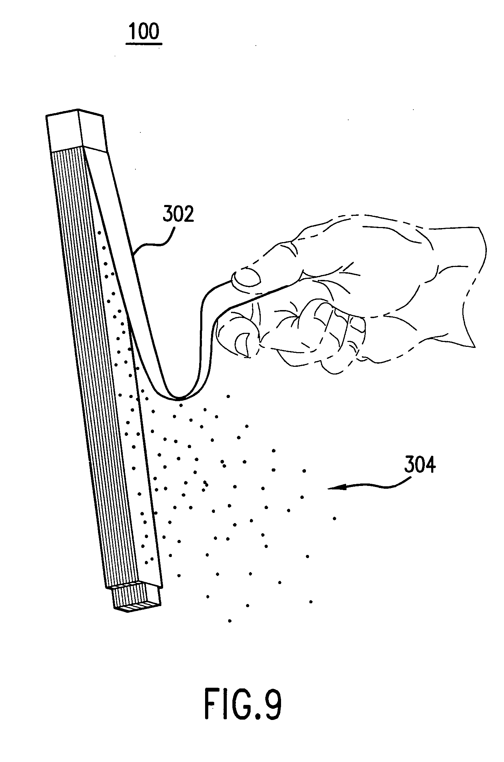

Figure 9 is an illustration of an embodiment of the present invention in use. As shown, a consumer may lift sealant layer 302 by tab 104 and remove sealant layer 302 from apparatus 100. As sealant layer 302 is removed, first scented layer 304 may release fragrance. First and second scented layer 304, 306 may release fragrance as they evaporate.

In an alternate embodiment, components of first and second layers 304, 306 may include chemicals that release fragrance upon exposure to air. This may include chemicals that react to air or any compounds in air, e.g., moisture, oxygen, etc. Alternatively, components of first and second layers 304, 306 may include chemicals that react with each other to release fragrance upon the physical action of removing sealant layer 302. Or, components of first and second layers 304, 306 may include chemicals that react on a delayed or time-release basis, thereby metering fragrance release.

In another alternate embodiment, a heating device may replace sealant layer 302 in apparatus 100. The heating device may be used to heat first and second scented layers 304, 306. Upon heating, layers 304, 306 may release fragrance.

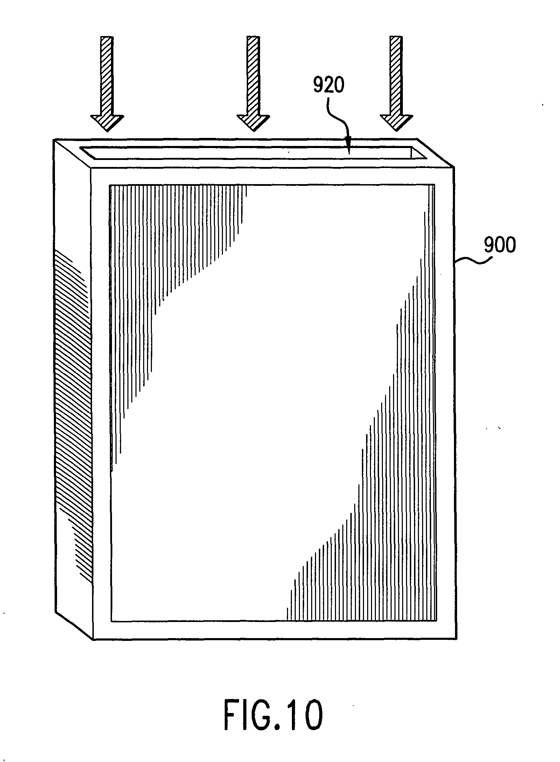

Figure 10 is an illustration of an exemplary container that may house an embodiment of the present invention. Container 900 may be at least of a size such that apparatus 100 may slide into, clip onto, be adhered to, or in some manner be included within container 900 through opening 920. Container 900 is not limited to any particular form, color, intensity of colors, or set of graphics. Container 900 may advantageously provide easy transport of apparatus 100. Additionally, container 900 may be used as a dispenser for apparatus 100 such that fragrance releasing units 102 may be easily dispensed.

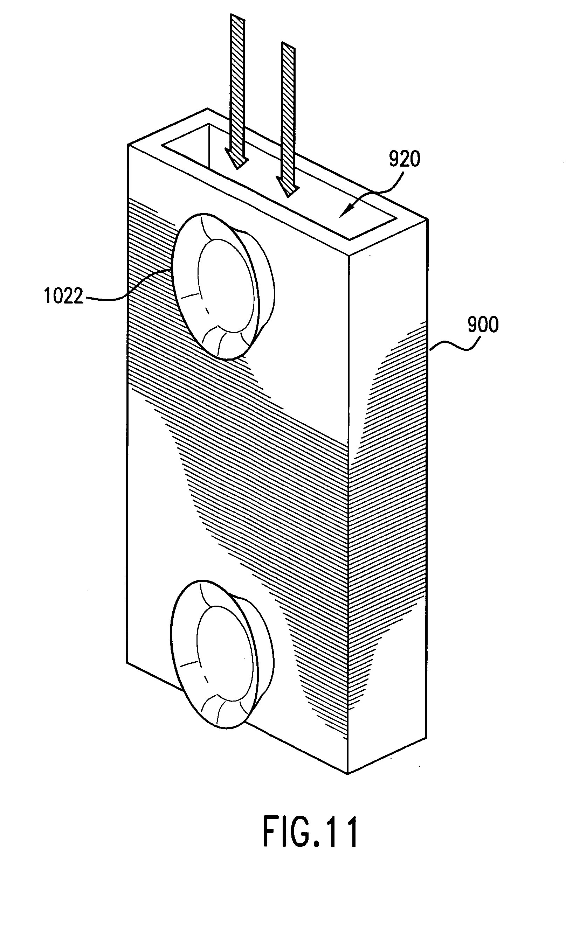

Figure 11 is an illustration of a side view of the exemplary container of Figure 9. Container 900 may be mounted on any surface using mounting devices 1022. Mounting devices 1022 may include, but is not limited to, a hanger hole, magnetic backing, adhesive backing, Velcro, a hanging hook, devices for adhering or clipping apparatus 100 to air vents, both out flow and intake, etc. Mounting devices 1022 may be attached to the back of container 900 or, alternatively, to apparatus 100 itself. Mounting advantageously allows embodiments of the present invention to be placed almost anywhere.

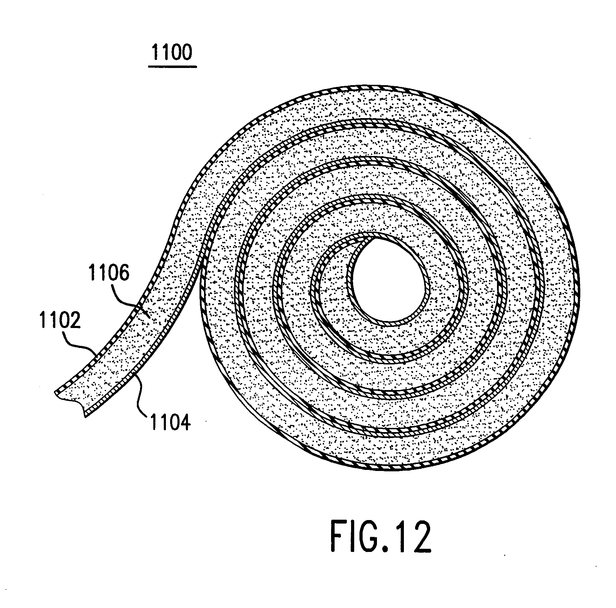

Figure 12 is a side view of an embodiment of a rolled fragrance releasing apparatus according to the present invention. Roll 1100 may include a sealant layer 1102, a first scented layer 1104, and a second scented layer 1106. The continuous roll 1100 may create outer layers that surround inner layers, thereby sealing the inner layers. Accordingly, sealant layer 1102 may form the outer layer of the roll and seal in scented layers 1104, 1106 in order to preserve the fragrance until the consumer is ready to use roll 1100. Fragrance may be released by unrolling roll 1100 to expose first scented layer 1104 to the air. After scented layers 1104, 1106 are depleted, sealant layer 1102 may be discarded.

Sealant layer 1102 may be made of plastic, plastic-like material, polymer, polymer-like material, modified paper sheets or any other material capable of sealing in at least a gel, solid, and/or some other substance form. Layer 1102 may also be biodegradable and/or scented. First scented layer 1104 may release a portion of fragrance from roll 1100. Layer 1104 may be an aroma-containing gel, solid, or similar volatile substance. Second scented layer 1106 may release another portion of fragrance from roll 1100 after first layer 1104 is depleted. Layer 1106 may be an aroma-containing gel, solid, or similar volatile substance.

In an alternate embodiment, sealant layer 1102 may be removed from second scented layer 1106, exposing second scented layer 1106. Second scented layer 1106 may then release fragrance followed by first scented layer 1104. After first scented layer 1104 is depleted, sealant layer on that portion of roll 1100 underneath may be exposed.

Roll 1100 may be made up of perforated sheets. As such, each sheet may include a unit of sealant layer 1102, first scented layer 1104, and second scented layer 1106. A sheet may be detached from roll 1100 by pulling along the perforation. Or roll 1100 may be made up of separate sheets, such that the sheets including layers 1102, 1104, 1106 may be placed side by side and rolled to form roll 1100. This sheet may be detached from roll 1100 by simply pulling the sheet from the roll. Alternatively, roll 110 may include a backing sheet onto which separate units of layers 1102, 1104, 1106 may be placed and rolled to form roll 1100. In this case, a unit may be detached from roll 1100 by peeling the unit from the backing sheet. Additional configurations of roll 1100 may be possible to separate the desired section of the fragrance releasing apparatus from the roll.

Roll 1100 may be attached to a dispensing device, such as a roller, a holder, etc., in order to easily unroll and dispense the fragrance releasing units. The dispensing device may be mountable on a surface or free standing, for example.

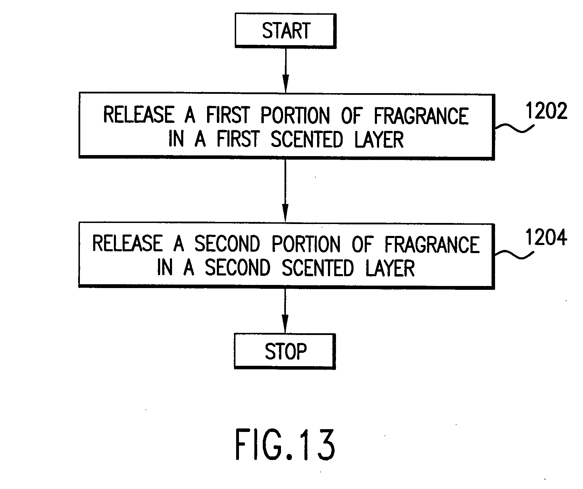

Figure 13 is a method for releasing fragrance according to an embodiment of the present invention. A first scented layer may be disposed on or adjacent to a second scented layer. A first portion of fragrance may be released (1202) from the first scented layer. The rate of release may be fast, moderate, or slow. After the first scented layer is depleted, the second scented layer may be exposed. The second scented layer may then release (1204) a second portion of fragrance. The rate of release may be fast, moderate, or slow. The rates of release for the two layers may be the same or different. And the fragrances in the two layers may be the same or different.

In an embodiment of a plurality of stacked units, after the second scented layer is depleted, the first scented layer of the unit underneath may be exposed. The method of Figure 13 may then be repeated for the unit underneath and the next and so on.

In an alternate method, the apparatus may include a top sealant layer covering at least the first scented layer. So, first, the top sealant layer may be removed from the apparatus, exposing the first scented layer. The first scented layer may then release the first portion of fragrance. In an embodiment of a plurality of stacked units having top sealant layers, after the second scented layer is depleted, the sealant layer of the unit underneath may be exposed. This alternate method may then be repeated for the unit underneath and the next and so on.

In another alternate method, the apparatus may also include a bottom sealant layer adjacent to the second scented layer. So, after the second scented layer is depleted, the bottom sealant layer may be exposed and then discarded. In an embodiment of a plurality of stacked units having bottom sealant layers, the bottom sealant layer may be removed from the apparatus, exposing the top sealant layer of the unit underneath. The method may then be repeated for the unit underneath and the next and so on.

The above is a detailed discussion of the preferred embodiments of the invention. The full scope of the invention to which applicants are entitled is defined by the claims hereinafter. It is intended that the scope of the claims may cover other embodiments than those described above and their equivalents.

## Claims

1. An apparatus for releasing fragrance, comprising:
a first layer of material configured to release a first portion of fragrance; and
a second layer of material adjacent to the first layer and configured to release a second portion of fragrance,
the second layer configured for releasing the second portion of fragrance after the first layer releases the first portion of fragrance.

2. The apparatus of claim 1, further comprising:
a third layer of material removably covering at least the first layer and configured to seal in fragrance, the first layer configured for releasing the first portion of fragrance after the third layer is removed from covering the first layer.

3. The apparatus of claim 2, further comprising:
side sealant portions covering the sides of the first and second layers;
end sealant portions covering the ends of the first and second layers; and
a fourth layer adjacent to the second layer removably connected thereto and cooperating with the side and end sealant portions and the third layer to complete encapsulation of the first and second layers until the third layer is removed.

4. The apparatus of any of claims 1 to 3, wherein the ratio of the first portion of fragrance to the second portion of fragrance results in a quick release of the first portion followed by a slow release of the second portion.

5. The apparatus of any of claims 1 to 3, wherein the ratio of the first portion of fragrance to the second portion of fragrance results in the release of a strong aroma of the first portion followed by the release of a weaker aroma of the second portion.

6. The apparatus of any of claims 1 to 3, wherein the ratio of the first portion of fragrance to the second portion of fragrance results in the release of a weak aroma of the first portion followed by the release of a strong aroma of the second portion.

7. The apparatus of any of claims 1 to 3, wherein the ratio of the first portion of fragrance to the second portion of fragrance results in the release of an aroma of the first portion followed by the release of an aroma of the second portion, the aromas being equal strength.

8. The apparatus of any of claims 1 to 7, wherein the first and second portions are the same fragrance.

9. The apparatus of any of claims 1 to 7, wherein the first and second portions are different fragrances.

10. The apparatus of any of claims 1 to 9, further comprising:
a heating device coupled to the first and second layers and configured to heat the first and second layers, wherein the first portion of fragrance and the second portion of fragrance are released after heating.

11. The apparatus of any of claims 1 to 10, wherein the first portion of fragrance and the second portion of fragrance are released on a time-release basis.

12. The apparatus of any of claims 1 to 11, wherein the first and second layers include chemicals that react with each other to release the respective first and second portions of fragrance.

13. The apparatus of any of claims 1 to 12, wherein the first and second layers react with at least one compound in air in order to release the respective first and second portions of fragrance.

14. The apparatus of any of claims 1 to 13, wherein the first and second layers evaporate in order to release the respective first and second portions of fragrance.

15. The apparatus of any of claims 1 to 14, further comprising:
an aromatic layer disposed on the first layer and configured to release a third portion of fragrance prior to the release of the first portion of fragrance from the first layer.

16. The apparatus of any of claims 1 to 15, wherein the apparatus forms a roii, the second layer being an outer layer and the first layer being disposed on the interior surface of the second layer.

17. The apparatus of any of claims 1 to 16, wherein the first and second layers have at least one of graphics and words embedded therein.

18. An apparatus for releasing fragrance, comprising:
a plurality of adjacent units, each unit including
a first layer configured to release a first portion of fragrance,
a second layer adjacent to the first layer and configured to release a second portion of fragrance,
a first sealant removably covering at least the first layer, and
a second sealant removably connected to the first sealant and configured to complete encapsulation of the first and second layers,
wherein the first layer releases the first portion after the first sealant is removed from covering the first layer and the second layer releases the second portion of fragrance after the first layer releases the first portion of fragrance.

19. The apparatus of claim 18, further comprising:
a mounting device detachably connected to the second sealant for mounting the apparatus to a surface.

20. The apparatus of claim 18 or 19, further comprising:
a container for housing the apparatus, the container being mountable on a surface.

21. The apparatus of any of claims 18 to 20, wherein the units are arranged in a stacked relationship such that fragrance in subsequent units is not released until a preceding unit has been removed from the apparatus.

22. A method for releasing fragrance from a fragrance releasing unit, comprising:
releasing a first portion of fragrance in a first scented layer of the unit; and
releasing a second portion of fragrance in a second scented layer of the unit,
wherein the first scented layer is disposed on the second scented layer and the second scented layer releases the second portion of fragrance after the first scented layer releases the first portion of fragrance.

23. The method of claim 22, further comprising:
removing a first sealant from the unit, the first sealant removably covering at least the first scented layer, the first scented layer releasing the first portion of fragrance after the first sealant is removed from the first layer.

24. The method of claim 22 or 23, further comprising:
removing a second sealant of the unit after the releasing of the second portion of fragrance.

25. The method of any of claims 22 to 24, wherein the releasing the first portion comprises quickly evaporating the first portion of fragrance and wherein the releasing the second portion comprises slowly evaporating the second portion of fragrance.

26. The method of any of claims 22 to 25, wherein the releasing the first portion comprises releasing the first portion of fragrance on a time-release basis and the releasing the second portion comprises releasing the second portion of fragrance on a time-release basis.

27. The method of any of claims 22 to 26, further comprising:
embedding at least one of graphics and words in the first and second scented layers.

28. A method for releasing fragrance from a plurality of fragrance releasing units, comprising:
releasing a first portion of fragrance in a first of the plurality of fragrance releasing units;
when the first portion decreases below a predetermined concentration, releasing a second portion of fragrance from a second of the plurality of fragrance releasing units,
wherein the first unit is disposed on the second unit.

29. The method of claim 28, wherein the releasing of the first portion of fragrance includes:
releasing the first portion of fragrance from a first scented layer and a second scented layer; and
removing a sealant layer from covering the first and second scented layers in order to release the first portion.

30. The method of claim 28, wherein the releasing the second portion of fragrance includes:
releasing the second portion of fragrance from a first scented layer and a second scented layer; and
removing a sealant layer from covering the first and second scented layers in order to release the second portion.

31. A method for transferring fragrance from a fragrance releasing unit, comprising:
removing a sealant from the fragrance releasing unit;
exposing a portion of fragrance in a scented layer of material in the unit; and
transferring the exposed portion of fragrance to a consumer.

32. The method of claim 31, wherein the transferring includes:
transferring the exposed portion of fragrance to the consumer's skin.

33. The method of claim 31, further comprising:
releasing the exposed portion of fragrance upon contact with the consumer.

34. The method of claim 31, further comprising:
carrying the unit in the consumer's purse, wallet, or pocket.

35. An apparatus for releasing fragrance, comprising:
a fragrance dispensing unit consisting of a plurality of sealed layers, the layers disposed one on top of another.

36. The apparatus of claim 35, wherein the plurality of sealed layers includes:
a first layer of material configured to release a first portion of fragrance; and
a second layer of material adjacent to the first layer and configured to release a second portion of fragrance,
the second layer configured for releasing the second portion of fragrance after the first layer releases the first portion of fragrance.

37. The apparatus of claim 35 or 36, wherein the plurality of sealed layers includes:
a third layer of material removably covering at least the first layer and configured to seal in fragrance, the first layer configured for releasing the first portion of fragrance after the third layer is removed from covering the first layer.

38. The apparatus of claim 36 or 37, wherein the plurality of sealed layers includes:
an aromatic layer of material disposed on the first layer and configured to release a third portion of fragrance prior to the release of the first portion of fragrance from the first layer.

39. The apparatus of any of claims 35 to 38, wherein the fragrance dispensing unit forms a stack.

40. The apparatus of any of claims 35 to 38, wherein the fragrance dispensing unit forms a roll.

41. The apparatus of claim 40, wherein the roll is perforated.

42. The apparatus of claim 40 or 41, wherein the roll includes separate sheets of the fragrance releasing unit.

43. The apparatus of any of claims 40 to 42, wherein the roll includes a backing sheet onto which separate sheets of the fragrance releasing unit are placed.

44. The apparatus of any of claims 35 to 43, further comprising:
a dispensing device coupled to the fragrance releasing unit to dispense sections of the fragrance releasing unit.
